# EUROPEAN PATENT APPLICATION

(11) **EP 4 477 660 A1**
(43) Date of publication of application: **18.12.2024**
(21) Application number: 23753062.1
(22) Date of filing: 02.02.2023
(51) Int. Cl.: C07K 7/64, G01N 33/574, A61K 38/00, A61P 35/00, A23L 33/18, A61K 47/42

(54) **PEPTIDE BINDING TO MESOTHELIN, AND USE THEREOF**

(30) Priority: 10.02.2022 KR 20220017283; 01.02.2023 KR 20230013822
(71) Applicant: Kyungpook National University Industry-Academic Cooperation Foundation, Daegu 41566 (KR)
(72) Inventor: LEE, Byung-Heon, Daegu 42038 (KR); PARK, Min-Sung, Daegu 41728 (KR)
(74) Representative: Mewburn Ellis LLP
(86) International application number: PCT/KR2023/001497
(87) International publication number: WO 2023/153711

(57) **Abstract**

The present invention relates to a peptide binding to mesothelin, and use thereof, wherein the peptide of the present invention specifically binds to mesothelin and inhibits same, and thus the effect of inhibiting the migration and metastasis of cancer cells is exhibited. It has been identified that the peptide of the present invention was selected, using a phage peptide display technology, as one type of peptide that easily binds to cells in which human mesothelin is highly expressed, and thus the peptide binds to mesothelin and inhibits the migration of cancer cells. The peptide of the present invention is relatively stable in serum, and thus exhibits high potential as an anti-cancer therapeutic agent for inhibiting metastasis according to cancer progression and migration in the future, an anti-cancer drug delivery probe, and a cancer diagnostic probe.

## Description

### Technical Field

The present disclosure relates to a peptide binding to mesothelin and a use for diagnosis and treatment of cancer and inhibition of cancer metastasis using the same.

### Background Art

Pancreatic cancer, which is the leading cause of cancer-related deaths worldwide, is difficult to diagnose before onset of widespread local invasion and metastasis to distant organs. In addition, many patients diagnosed with pancreatic cancer have symptoms that are not directly related to cancer, such as anorexia, malabsorption, and vomiting, making it difficult to diagnose and thus resulting in worsening of the prognosis. So far, there are no absolute tools capable of effectively diagnosing pancreatic cancer, and the mortality rate of pancreatic cancer is higher than that of other cancers due to its strong resistance to chemotherapy. Surgical resection is the main treatment for pancreatic cancer, but the postoperative prognosis in patients is poor.

Mesothelin, a protein with a molecular weight of about 40 kDa, is overexpressed in a variety of solid tumors, particularly mesothelioma, pancreatic, ovarian, lung, and biliary tract cancers. In the pancreas, mesothelin has been found predominantly in pancreatic cancer and showed tumor specificity that is not expressed in normal or chronic pancreatitis. While pancreatic cancer develops through mutations or malfunction of many genes and proteins through various stages, mesothelin mostly increases in number in advanced stages, i.e., invasive pancreatic cancers. Mesothelin is considered to play a role in the adhesion of cells, but its function is still unknown. What is known to date is that overexpression of mesothelin increases the invasion and migration of pancreatic cancer. On pancreatic cancer cell membranes, the binding between MUC16 and mesothelin activates MAPK p38 and stimulates MMP7 synthesis, thereby increasing its invasiveness and migratability. With no MUC16 expression in pancreatic cancer cell membranes, mesothelin randomly activates the ERK pathway to increase MMP7 expression. Overexpression of mesothelin increases STAT3 activation and expression of cyclin E, which then affects proliferation. In addition, activated AKT triggers activation of NF-κB as well as an increase in IL-6 expression and finally affects proliferation and survival, leading to an increase in the anti-apoptotic proteins Bcl-XL and Mcl-1 and a decrease in the apoptotic proteins BAX and BAD. From the above report, it may be noticed that the function of mesothelin increases proliferation, survival and invasion of pancreatic cancer. In addition, there is a significant association between short survival in pancreatic cancer patients and overexpression of mesothelin. Therefore, detection and regulation of mesothelin will be useful in the diagnosis and treatment of pancreatic cancer, and the development of peptides that bind specifically to mesothelin and regulate the binding will be a useful tool in the diagnosis and treatment of pancreatic cancer.

### Disclosure of the Invention

### Technical Goals

The present disclosure relates to a peptide binding to mesothelin and a use thereof, specifically, an object of the present disclosure is to provide a peptide for specifically binding to mesothelin, comprising an amino acid sequence represented by SEQ ID NO: 1, a composition for diagnosing cancer, pharmaceutical composition for inhibiting cancer metastasis, composition for drug delivery, and composition for cancer cell imaging including the peptide as an active ingredient; and a fusion peptide in which the peptide and an apoptotic peptide comprising an amino acid sequence represented by SEQ ID NO: 2 are linked, a pharmaceutical composition for preventing or treating cancer including the fusion peptide as an active ingredient, and a pharmaceutical composition for preventing or treating cancer including the fusion peptide and an anticancer agent as active ingredients.

### Technical Solutions

To achieve the above object, the present disclosure provides a peptide for specifically binding to mesothelin, comprising an amino acid sequence represented by SEQ ID NO: 1.

In addition, the present disclosure provides a polynucleotide encoding the peptide, a recombinant vector including the polynucleotide, and a transformant transformed with the recombinant vector.

In addition, the present disclosure provides a composition for diagnosing cancer, including the peptide as an active ingredient.

In addition, the present disclosure provides a pharmaceutical composition for inhibiting cancer metastasis, including the peptide as an active ingredient.

In addition, the present disclosure provides a composition for drug delivery, including the peptide as an active ingredient.

In addition, the present disclosure provides a fusion peptide in which the peptide and an apoptotic peptide comprising an amino acid sequence represented by SEQ ID NO: 2 are linked.

In addition, the present disclosure provides a pharmaceutical composition for preventing or treating cancer, including the fusion peptide as an active ingredient.

In addition, the present disclosure provides a pharmaceutical composition for preventing or treating cancer, including the fusion peptide and an anticancer agent as active ingredients.

In addition, the present disclosure provides a composition for cancer cell imaging, including the peptide as an active ingredient.

### Advantageous Effects

The present disclosure relates to a peptide binding to mesothelin and a use thereof, wherein the peptide of the present disclosure specifically binds to mesothelin and inhibits the mesothelin, thereby exhibiting an effect of suppressing migration and metastasis of cancer cells. The peptide of the present disclosure is one selected type of peptides binding well to cells that have high expression levels of human mesothelin using a phage peptide display technology, and it was determined that the peptide binds to mesothelin and suppresses migration of cancer cells. The peptide of the present disclosure is relatively stable in serum and shows high potential for the future as an anticancer treatment agent, probe for anticancer drug delivery, and cancer diagnostic probe capable of inhibiting metastasis due to progression and migration of cancer.

### Brief Description of Drawings

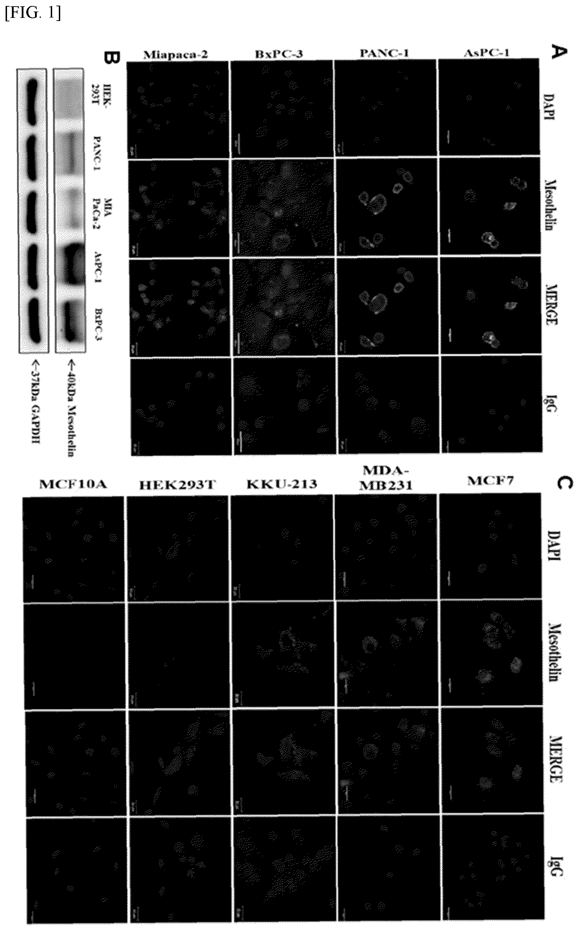

FIG. 1 shows expression of mesothelin in pancreatic cancer cell lines using mesothelin antibodies. (A) Results of identifying expression of mesothelin in pancreatic cancer cell lines AsPC-1, PANC-1, BxPC-3, and MiaPaCa-2 via immunofluorescence analysis. (B) Results of identifying expression of mesothelin in pancreatic cancer cell lines AsPC-1, PANC-1, BxPC-3, and MiaPaCa-2 via Western blot assay. (C) Results of identifying expression of mesothelin in breast cancer cell lines MDA-MB231 and MCF7, a cholangiocarcinoma cell line KKU-213, a normal cell line HEK293T, and a normal breast cell line MCF10A via immunofluorescence analysis.

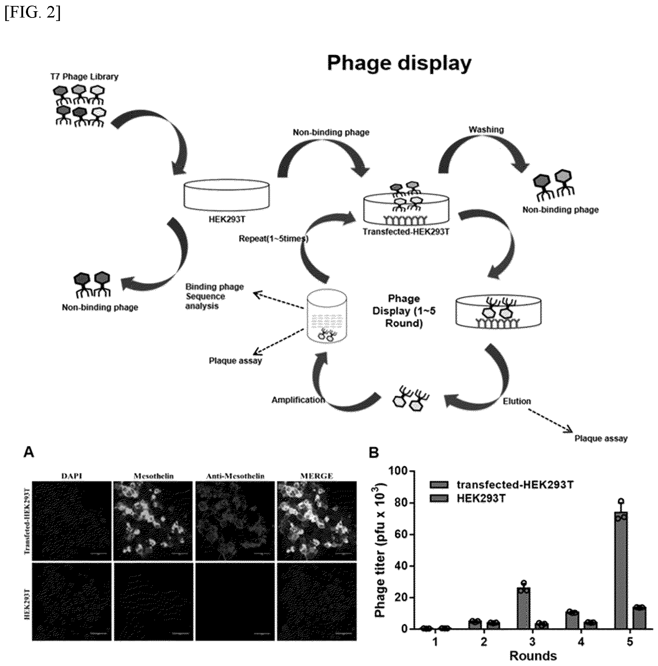

FIG. 2 shows results of screening peptides that bind to mesothelin using phage display. (A) Results of identifying whether mesothelin-expressing HEK293T cell lines transfected with DNA and untransfected cell lines express mesothelin via immunofluorescence analysis. (B) Results of 5 repetitive phage displays performed using mesothelin-expressing HEK293T cell lines transfected with DNA and untransfected cell lines.

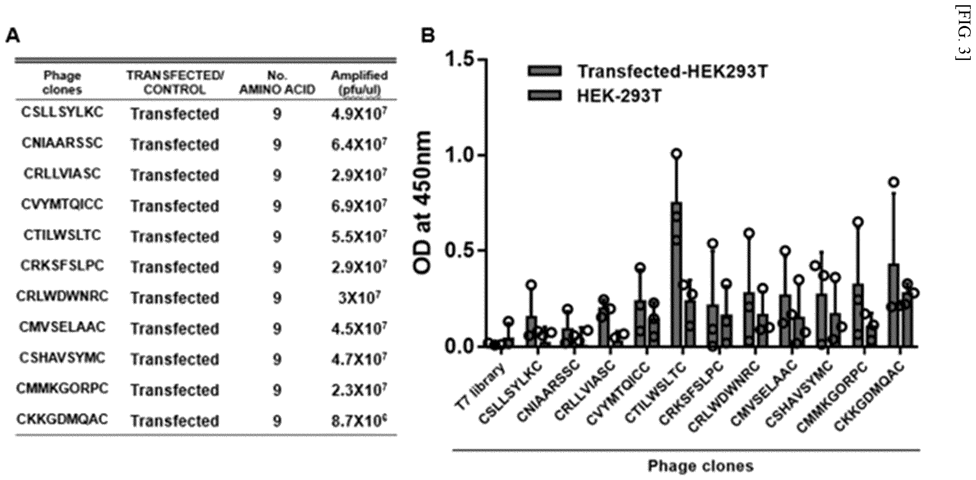

FIG. 3 shows results of phage ELISA analysis of 11 sequences extracted through phage display. (A) Results organized in Table for 11 sequences extracted after performing 5 repetitive phage display. (B) Results of measuring absorbance of 11 extracted sequences through phage ELISA analysis.

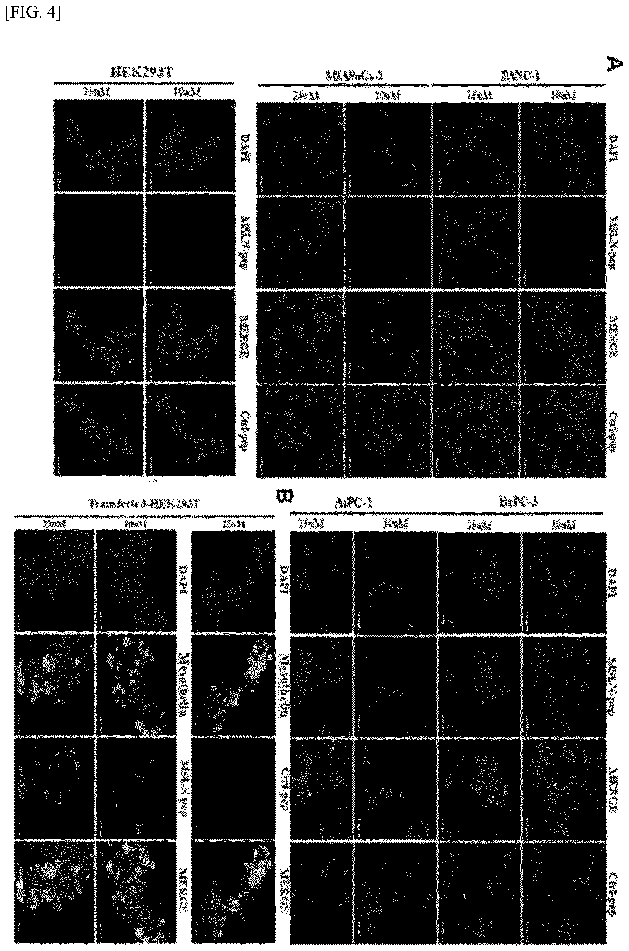

In FIG. 4, a CTILWSLTC sequence that specifically binds to mesothelin was synthesized in the form of peptide and then named MSLN-pep. (A) Results of observing TAMRA-labeled MSLN-pep in mesothelin-expressing pancreatic cancer cell lines AsPC-1, PANC-1, BxPC-3, and MiaPaCa-2 via immunofluorescence analysis. (B) Results of observing MSLN-pep in mesothelin-expressing HEK293T cell lines transfected with DNA via immunofluorescence analysis.

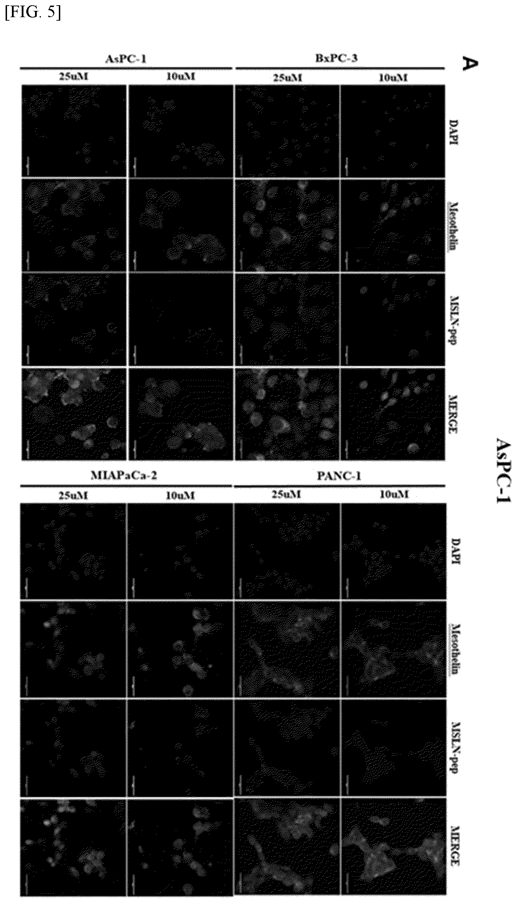

FIG. 5 shows results that mesothelin antibodies and MSLN-pep are expressed in the same spot. (A) Results of determining whether mesothelin antibodies and TAMRA-labeled MSLN-pep are stained in the same spot in pancreatic cancer cell lines AsPC-1, PANC-1, BxPC-3, and MiaPaCa-2 via co-localization analysis.

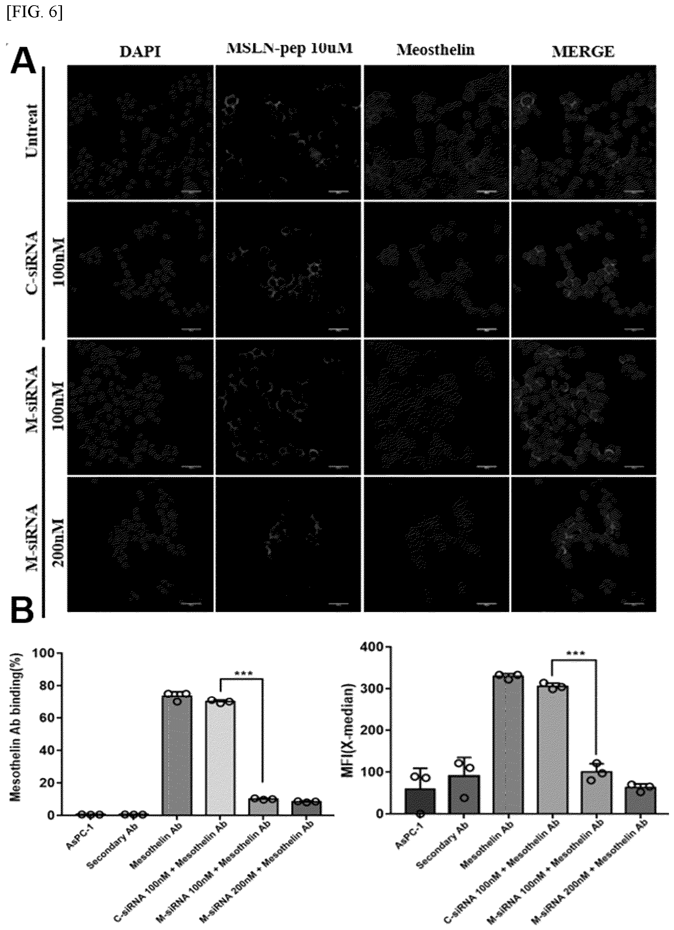

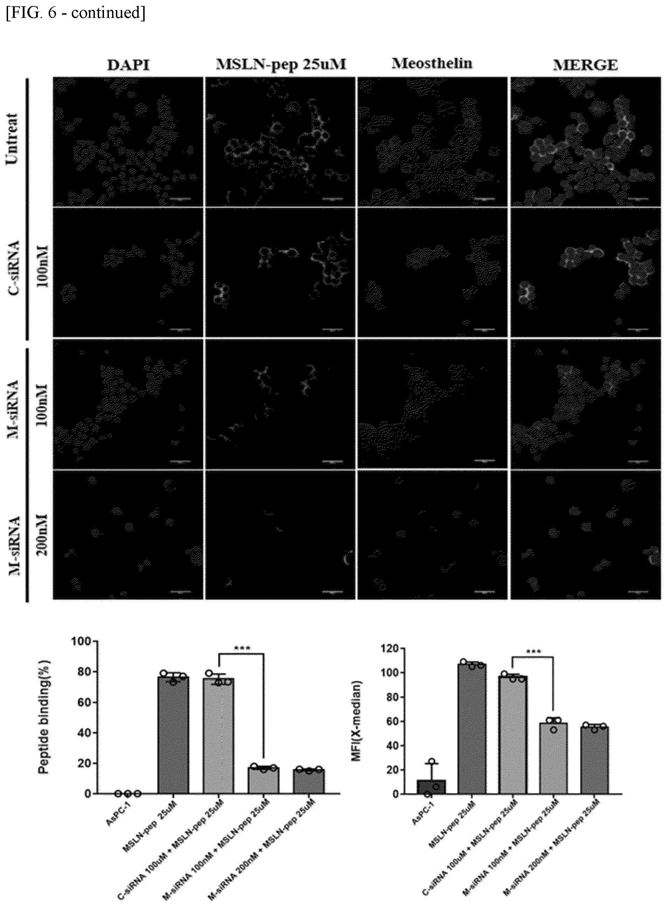

FIG. 6 shows results of binding between mesothelin antibodies and MSLN-pep after suppressing expression of mesothelin with siRNA in a mesothelin-expressing pancreatic cancer cell line AsPC-1. (A) Results of determining, via co-localization immunofluorescence analysis, whether mesothelin antibodies and FITC-labeled MSLN-pep are stained in the same spot after suppressing expression of mesothelin with siRNA in AsPC-1. (B) Results of analyzing, after suppressing expression of mesothelin with siRNA in AsPC-1, the fluorescent intensity in the binding between mesothelin antibodies and the fluorescent intensity in the binding between FITC-labeled MSLN-pep and cells.

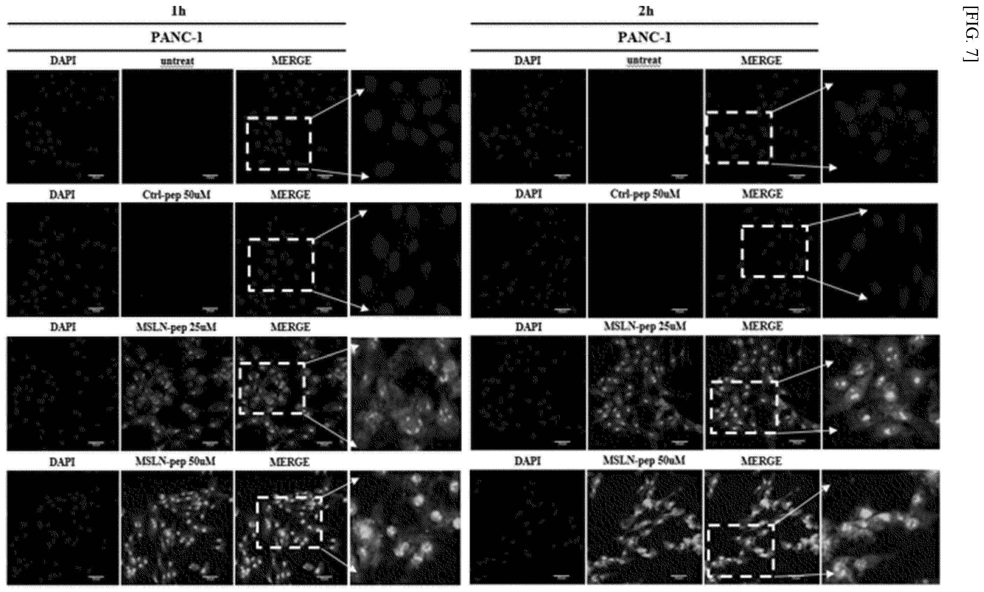

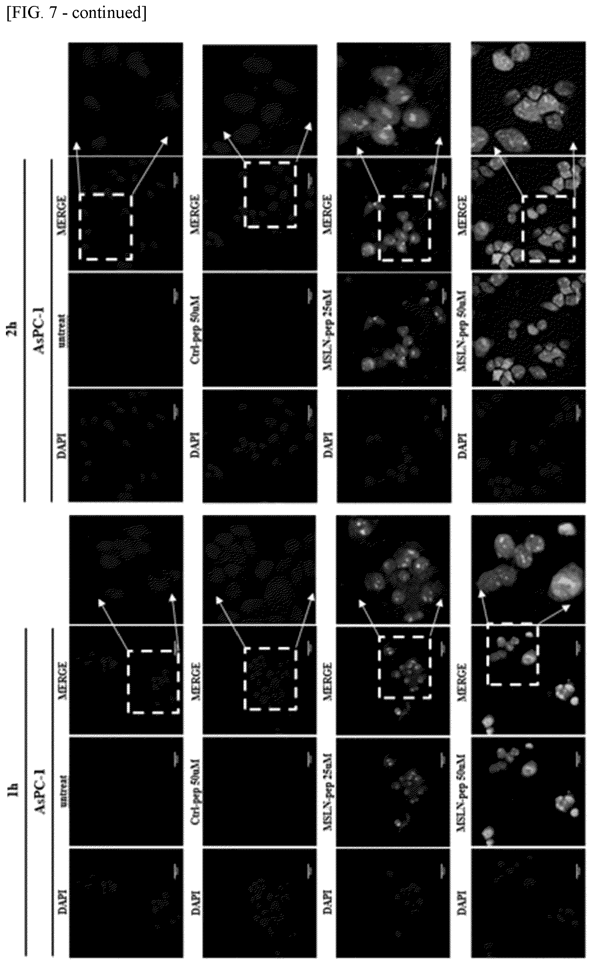

FIG. 7 shows internalization into cells after FITC-labeled MSLN-pep binds to pancreatic cancer cell lines PANC-1 and AsPC-1. (A) Results of identifying internalization into cells taking place 1 and 2 hours after binding FITC fluorescence reagent-labeled MSLN-pep to PANC-1 and AsPC-1 via immunofluorescence analysis.

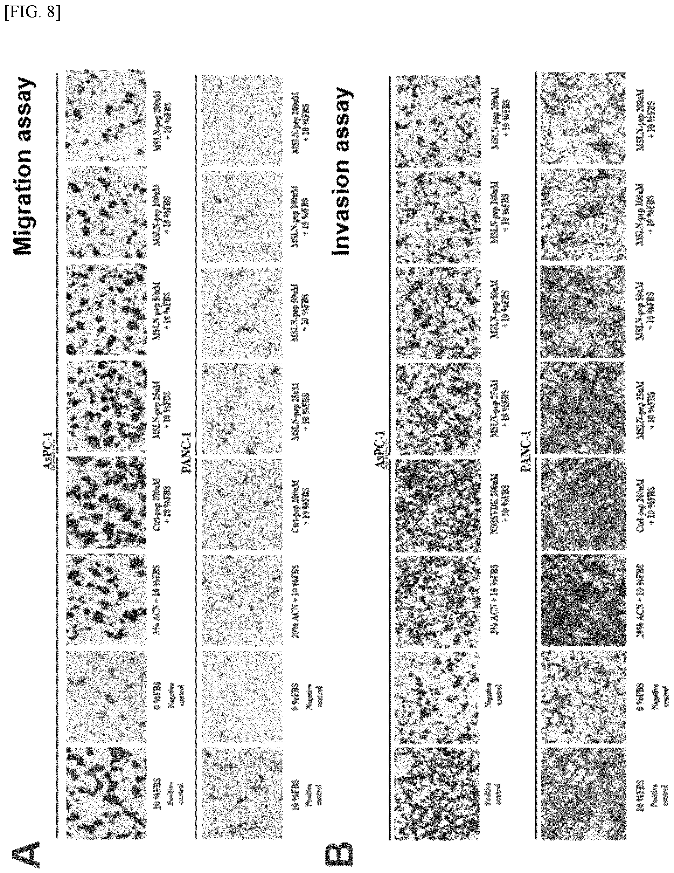

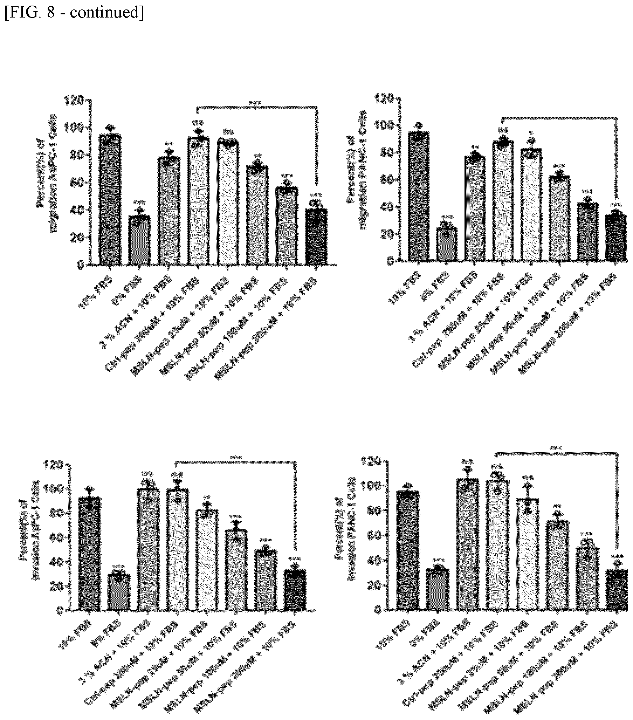

FIG. 8 shows results of analyzing cell migration and invasion after pancreatic cancer cell lines PANC-1 and AsPC-1 are treated with MSLN-pep. (A) Results of observing migration under fluorescence microscopy after treating PANC-1 and AsPC-1 with MSLN-pep, the results of which are shown in graph. (B) Results of observing invasion under fluorescence microscopy after treating PANC-1 and AsPC-1 with MSLN-pep, the results of which are shown in graph.

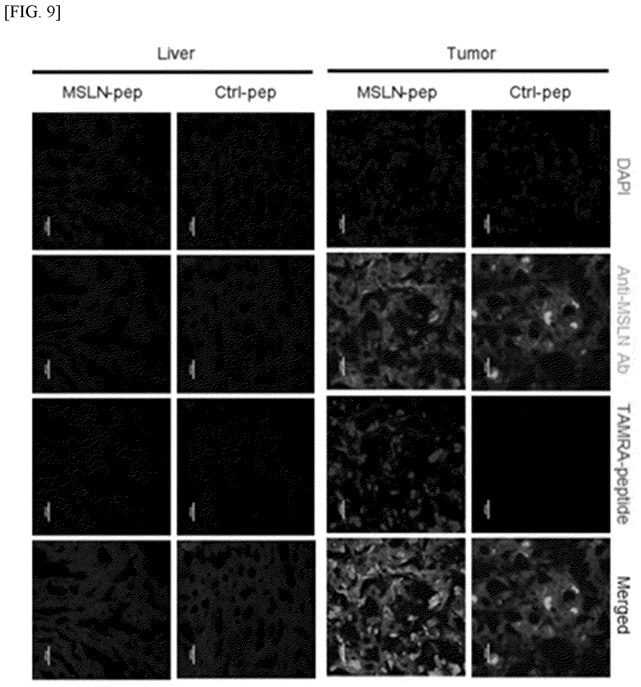

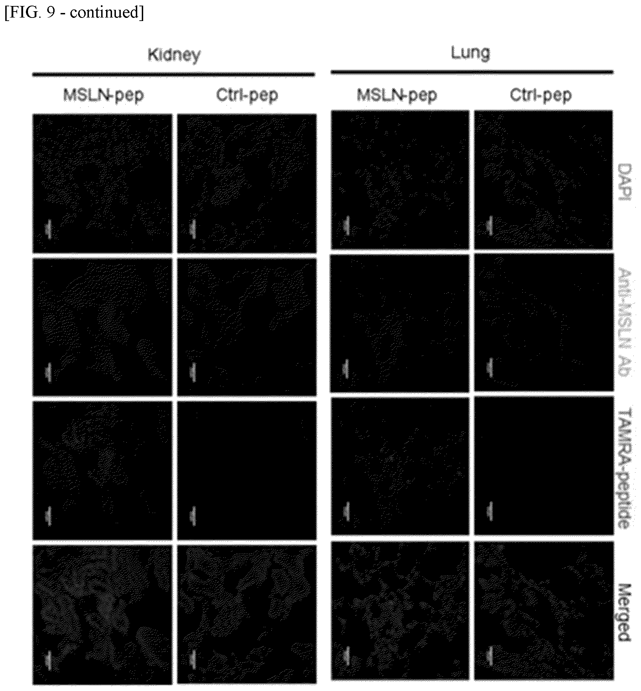

FIG. 9 shows results of observation under fluorescence microscopy after intravenously injecting MSLN-pep labeled with a tetramethylrhodamine (TAMRA) fluorescent reagent (red) and a control peptide (Ctrl-pep) into nude mice with tumor developed by implanting a pancreatic cancer cell line AsPC-1, separating tumor and control organs (liver, lung, and kidney) 6 hours later to prepare cryosections, and then carrying out antibody staining (green) for mesothelin. Here, nuclei of the cells were stained with a 4',6-diamidino-2-phenylindole (DAPI) reagent (blue).

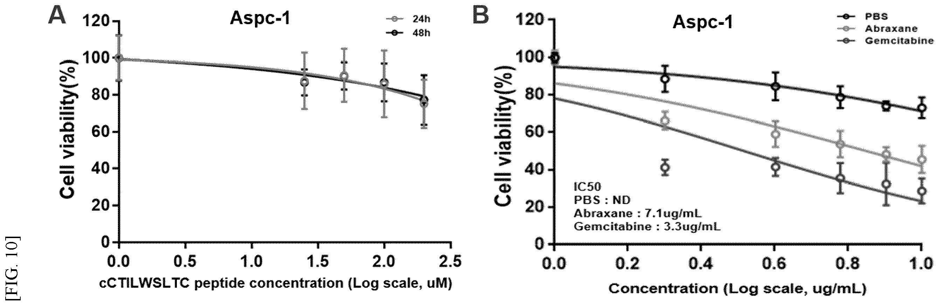

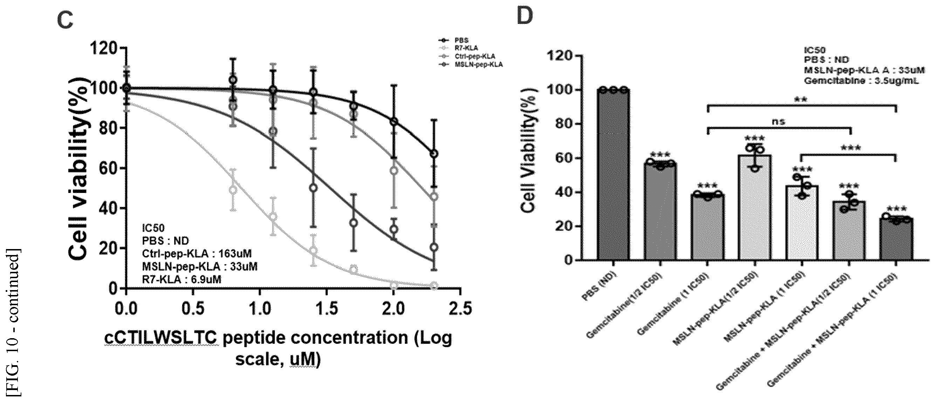

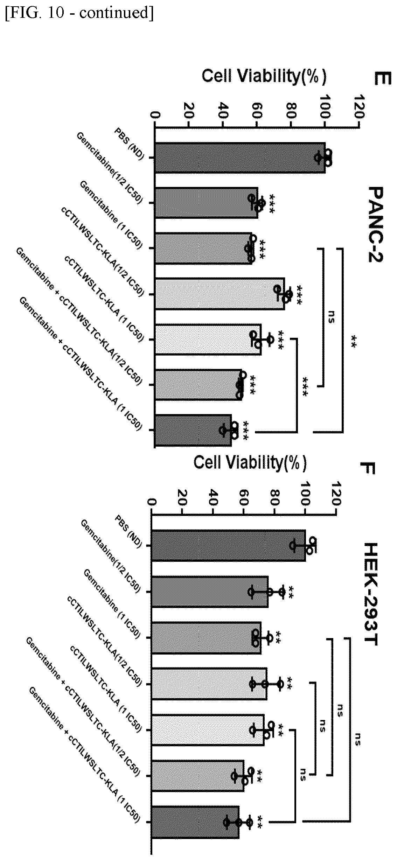

FIG. 10 shows results of comparison between cytotoxicity by treating a pancreatic cancer cell line AsPC-1 with MSLN-pep and cytotoxicity of anticancer agents Abraxane and gemcitabine after labeling MSLN-pep with apoptotic peptide KLAKLAKKLAKLAK. (A) Results of cytotoxicity when AsPC-1 is treated with MSLN-pep. (B) Results of cytotoxicity when AsPC-1 is treated with anticancer agents Abraxane and gemcitabine. (C) Results of cytotoxicity of MSLN-pep labeled with an apoptotic peptide KLAKLAKKLAKLAK in AsPC-1. (D) Results of cytotoxicity after treatment in a degree comparable to the IC50 value of MSLN-pep labeled with KLAKLAKKLAKLAK in AsPC-1 and the IC50 value of gemcitabine. (E) Results of cytotoxicity after treatment in a degree comparable to the IC50 value of MSLN-pep labeled with KLAKLAKKLAKLAK in a mouse pancreatic cancer cell line PANC-2 and the IC50 value of gemcitabine. (F) Results of cytotoxicity after treatment in a degree comparable to the IC50 value of MSLN-pep labeled KLAKLAKKLAKLAK to a normal cell line HEK293T that does not express mesothelin and the IC50 value of gemcitabine.

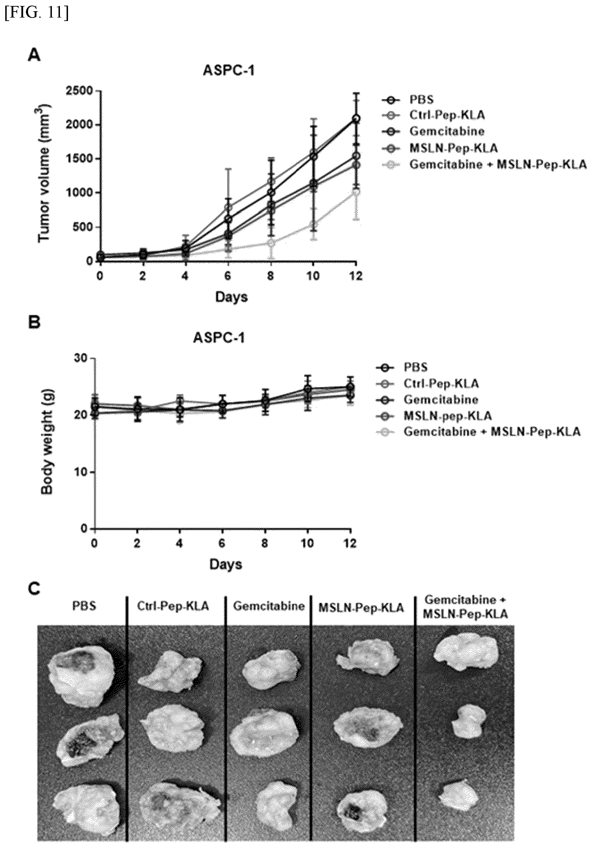

FIG. 11 shows results of investigating cancer growth inhibitory effects by treating nude mice with tumors developed by implanting a pancreatic cancer cell line AsPC-1 with MSLN-pep-KLA alone and in combination with gemcitabine. (A) A size of the tumor after treatment with MSLN-PEP-KLA alone and in combination with gemcitabine. (B) A body weight of mice after treatment with MSLN-pep-KLA alone and in combination with gemcitabine. (C) Tumors removed after the completion of chemotherapy.

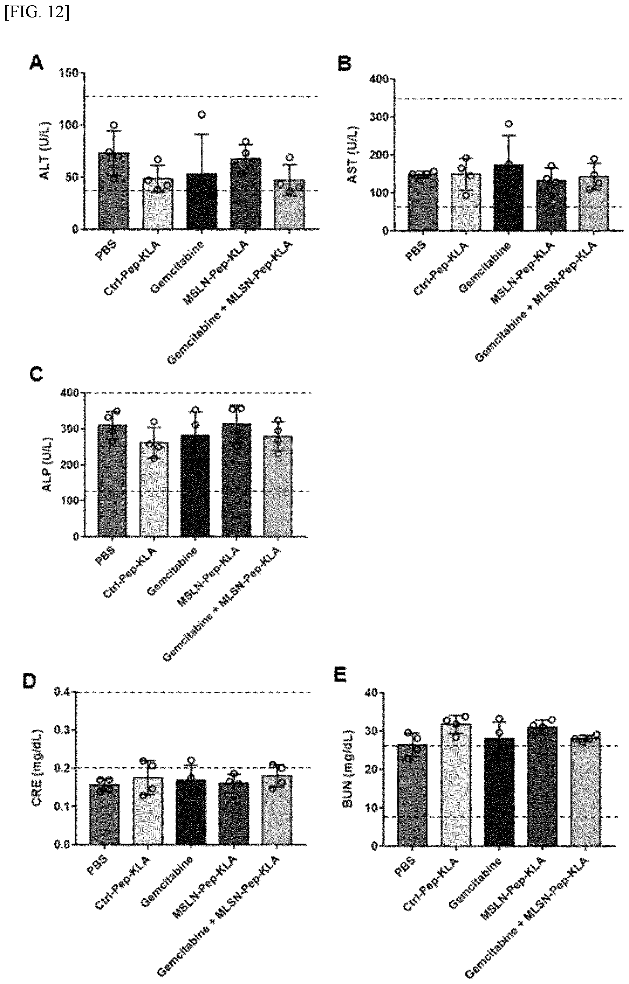

FIG. 12 shows results of investigating cancer growth inhibitory effects by treating nude mice with tumor developed by implanting a pancreatic cancer cell line AsPC-1 with MSLN-pep-KLA alone and in combination with gemcitabine. (A) A level of a liver enzyme ALT in the blood of mice after completion of chemotherapy. (B) A level of a liver enzyme AST. (C) A level of a liver enzyme ALP. (D) A level of creatinine (CRE) which is an indicator of kidney functions. (E) A level of BUN which is an indicator of kidney functions.

### Best Mode for Carrying Out the Invention

The present disclosure provides a peptide for specifically binding to mesothelin, comprising an amino acid sequence represented by SEQ ID NO: 1.

The amino acid sequence represented by SEQ ID NO: 1 in the present disclosure is "CTILWSLTC", abbreviated as "MSLN-pep" in the present specification.

The peptide of the present disclosure may be readily prepared by chemical synthesis known in the art. Typical methods include, but are not limited to, liquid or solid-phase synthesis, fragment condensation, and F-MOC or T-BOC chemical methods.

In addition, the peptide of the present disclosure may be prepared by a genetic engineering method. First, the DNA sequence encoding the peptide is synthesized according to conventional methods. DNA sequences may be synthesized by PCR amplification using appropriate primers. As other methods, DNA sequences may be synthesized using standard methods known in the art, e.g., automated DNA synthesizers (e.g., those sold by Biosearch or Applied Biosystems). The prepared DNA sequence is operatively linked to this DNA sequence and inserted into a vector including one or more expression control sequences (e.g., promoters and enhancers) that regulate the expression of that DNA sequence, and the host cell is transformed with a recombinant expression vector formed therefrom. The resulting transformant is cultured under appropriate media and conditions so that the DNA sequence is expressed, thereby harvesting the substantially pure peptide encoded by the DNA sequence from the culture. The harvesting may be performed using methods known in the art (such as chromatography). The term 'substantially pure peptide' as used herein refers to a state in which the peptide according to the present disclosure does not include substantially any other proteins derived from the host.

In the present disclosure, a peptide comprising an amino acid sequence represented by SEQ ID NO: 1 is a concept including a functional variant thereof. The term "functional variant" as used herein refers to any similar sequence in which substitution of some amino acids occurs at the loci of the amino acid without affecting the properties of the peptide of the present disclosure that binds specifically to mesothelin.

In addition, the present disclosure provides a polynucleotide encoding the peptide.

The "polynucleotide" as used herein refers to a polymer of deoxyribonucleotide or ribonucleotide existing in a single-stranded or double-stranded form. It encompasses RNA genome sequences, DNA (gDNA and cDNA), and RNA sequences transcribed therefrom and includes analogues of natural polynucleotides unless otherwise mentioned.

The polynucleotide includes not only the nucleotide sequence encoding the peptide, but also a sequence that is complementary to that sequence. The complementary sequence includes not only a perfectly complementary sequence, but also a substantially complementary sequence.

In addition, the polynucleotide may be modified. The modification includes the addition, deletion, or non-conservative substitution or conservative substitution of nucleotides. The polynucleotide encoding the amino acid sequence is interpreted to include the nucleotide sequence which also has substantial identity with respect to the nucleotide sequence. The substantial identity above may be at least 80% homology, at least 90% homology, or at least 95% homology shown in the sequence when the nucleotide sequence and any other sequences are complementarily aligned as much as possible, and the aligned sequences are analyzed using an algorithm commonly used in the art.

In addition, the present disclosure provides a recombinant vector including the polynucleotide.

In addition, the present disclosure provides a transformant (except for humans) transformed with the recombinant vector.

As used herein, the term "vector" refers to a self-replicating DNA molecule used to carry a clonal gene (or another piece of clonal DNA).

As used herein, the term "recombinant vector" refers to a plasmid, viral vector or other media known in the art that may express nucleic acid inserted into the host cell, wherein a polynucleotide, encoding the peptide of the present disclosure, may be operationally linked to a conventional expression vector known in the art. The recombinant vector may include an origin of replication capable of proliferation generally in the host cell, one or more expression regulatory sequences that regulate expression (e.g. promoter and enhancer), a selective marker, and a polynucleotide encoding a peptide of the present disclosure operably linked to an expression regulatory sequence. The transformant may be transformed with the recombinant vector.

Preferably, the transformant may be obtained by introducing a recombinant vector including a polynucleotide encoding the peptide of the present disclosure into the host cell by methods known in the art, such as, but not limited to, transient transfection, microinjection, transduction, cell fusion, calcium phosphate precipitation, liposome-mediated transfection, DEAE dextran-mediated transfection, polybrene-mediated transfection, electroporation, gene gun, and other known methods for introducing nucleic acids into the cell.

In addition, the present disclosure provides a composition for diagnosing cancer, including the peptide as an active ingredient.

Preferably, the cancer may be one with a high expression level of mesothelin, but is not limited to.

More preferably, the cancer may be, but is not limited to, mesothelioma, ovarian cancer, pancreatic cancer, lung cancer, cervical cancer, biliary tract cancer, breast cancer, head and neck cancer, or esophageal cancer.

As used herein, the term "diagnosis" refers to identification of the presence or characteristics of a pathological condition. For the purposes of the present disclosure, the diagnosis is to determine the presence or characteristics of cancer.

The diagnosis of cancer using the peptide of the present disclosure may be made by carrying out a reaction with the peptide of the present disclosure in the tissue or cell obtained directly from blood, urine or biopsy and then detecting their binding.

In addition, in order to easily identify, detect, and quantify whether the peptide of the present disclosure has bound to the cancerous tissue, the peptide of the present disclosure may be provided in the labeled form. In other words, it may be provided in a linked (e.g., covalently bonded or cross-linked) form to a detectable label. The detectable label may be chromogenic enzymes (e.g., peroxidase, alkaline phosphatase), radioisotopes (e.g., ¹²⁴I, ¹²⁵I, ¹¹¹In, ⁹⁹mTc, ³²P, and ³⁵S), chromophores, and luminescent or fluorescent substances (e.g., FITC, RITC, rhodamine, cyanine, Texas Red, fluorescein, phycoerythrin, and quantum dots).

Similarly, the detectable label may be antibody epitopes, substrates, cofactors, inhibitors, or affinity ligands. Such label may be performed in the process of synthesizing the peptide of the present disclosure, or it may be performed additionally on the already synthesized peptide. If fluorescent substance is used as a detectable label, fluorescence mediated tomography (FMT) may be used to diagnose cancer. For example, the peptide of the present disclosure labeled with the fluorescent substance may be circulated into the blood to observe the fluorescence by the peptide via fluorescence tomography. If fluorescence is observed, it is diagnosed as cancer.

In addition, the present disclosure provides a pharmaceutical composition for inhibiting cancer metastasis, including the peptide as an active ingredient.

Preferably, the peptide may bind specifically to mesothelin, be internalized into cancer cells, and inhibit the invasion and migration of the cancer cell, but is not limited thereto.

In addition, the present disclosure provides a composition for drug delivery, including the peptide as an active ingredient.

Preferably, the drug may be a peptide drug or an anticancer agent, and more preferably, the peptide drug may be a cytotoxic peptide with apoptosis or necrosis-inducing activity, but is not limited thereto.

The peptide according to the present disclosure may be used as an intelligent drug delivery that selectively delivers drugs to cancer cells. If the peptide of the present disclosure is used for cancer treatment by linking with a previously known drug, the efficacy of the drug may be increased because the drug is selectively delivered to the cancer cells by the peptide of the present disclosure, and at the same time, the side effects of the drug on the normal tissue may be significantly reduced.

The drug is an anticancer agent, and anything that is used for conventional cancer treatment may be used without limitation as an anticancer agent that may be linked to the peptide of the present disclosure. For example, the drug may include gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, or nitrosourea. The linkage between the anticancer agent and the peptide of the present disclosure may be carried out by methods known in the art, e.g., covalent bonds and cross-linking. To this end, the peptide of the present disclosure may, if necessary, be chemically modified to the extent in which its activity is not lost.

In addition, the present disclosure provides a fusion peptide in which the peptide and an apoptotic peptide having an amino acid sequence represented by SEQ ID NO: 2 are linked.

The apoptotic peptide comprising an amino acid sequence represented by SEQ ID NO: 2 is "KLAKLAKKLAKLAK", abbreviated herein as "KLA".

In the present disclosure, the fusion peptide is in the form in which the apoptotic peptides KLAKLAKKLAKLAK (KLA; SEQ ID NO: 2) and CTILWSLTC (MSLN-pep; SEQ ID NO: 1) are linked, abbreviated herein as "MSLN-pep-KLA".

In addition, the present disclosure provides a pharmaceutical composition for preventing or treating cancer, including the fusion peptide as an active ingredient.

In addition, the present disclosure provides a pharmaceutical composition for preventing or treating cancer, including the fusion peptide and an anticancer agent as active ingredients.

Preferably, the anticancer agent may include, but is not limited to, gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, or nitrosourea.

Preferably, the cancer may be a cancer whose expression levels of mesothelin are high, but is not limited to.

More preferably, the cancer may be, but is not limited to, mesothelioma, ovarian cancer, pancreatic cancer, lung cancer, cervical cancer, biliary tract cancer, breast cancer, head and neck cancer, or esophageal cancer.

The pharmaceutical composition of the present disclosure may be prepared using pharmaceutically suitable and physiologically acceptable adjuvants in addition to the active ingredient, and solubilizers such as excipients, disintegrating agents, sweeteners, binders, coating agents, swelling agents, lubricants, glydents, or flavoring agents may be used as the adjuvant. The pharmaceutical composition of the present disclosure may preferably be formulated as a pharmaceutical composition by including one or more types of pharmaceutically acceptable carriers in addition to the active ingredient for administration. Acceptable pharmaceutical carriers for compositions to be formulated as liquid solutions are those that are sterile and suitable to the body and may be used by mixing saline, sterile water, Ringer's solution, buffered saline, albumin injection solution, dextrose solution, maltodextrin solution, glycerol, ethanol, and one or more of these components, and other conventional additives such as antioxidants, buffers, and bacteriostatic agents may be added as required. In addition, diluents, dispersants, surfactants, binders, and lubricants may be additionally added to prepare in the form of injectable formulations such as aqueous solutions, suspensions, and emulsions, pills, capsules, granules, or tablets.

The pharmaceutical formulation form of the pharmaceutical composition of the present disclosure may be granules, acids, coated tablets, tablets, capsules, suppositories, syrups, juices, suspensions, emulsions, drips, or injectable liquids, and sustained-release preparations of active compounds. The pharmaceutical composition of the present disclosure may be administered in a conventional manner through the intravenous, intra-arterial, intra-abdominal, intramuscular, intra-arterial, intra-peritoneal, intrasternal, percutaneous, nasal, inhaled, topical, rectal, oral, intraocular, or intradermal routes. The effective amount of the active ingredient of the pharmaceutical composition of the present disclosure refers to an amount required for the prevention or treatment of diseases. Thus, it may be adjusted depending on various factors, including the type of disease, the severity of the disease, the type and content of active ingredients and other components contained in the composition, the type of formulation and the patient's age, weight, general health status, sex and diet, time of administration, route of administration and secretion rate of the composition, duration of treatment, and concomitant drugs. Although not limited to, for example, in the case of adults, when administered once to several times a day, the composition of the present disclosure may be administered at a dose of 0.1 ng/kg ~ 10 g/kg in the case of a compound when administered once to several times a day.

In addition, the present disclosure provides a composition for cancer cell imaging, including the peptide as an active ingredient.

Preferably, the peptide may be labeled with, but is not limited to, chromogenic enzymes, radioisotopes, chromophores, luminescent substances, fluorescers, magnetic resonance imaging substances, superparamagnetic particles, or ultrasuper paramagnetic particles.

Cancer cell imaging and cancer diagnosis may be, but are not limited to, used not only for the initial diagnosis of cancer diseases, but also for monitoring of the progression, progress of treatment, and response to treatment agents. The peptide may be provided in a labeled state to facilitate identification, detection, and quantification of binding, as described above.

### Modes for Carrying Out the Invention

Hereinafter, the present disclosure will be described in more detail through examples. However, it will be apparent to those skilled in the art that these examples are intended to describe the present disclosure in more detail, and the scope of the present disclosure is not limited to the following examples according to the gist of the present disclosure.

### <Experimental Example>

The following experimental examples are intended to provide experimental examples that are commonly applied to each examples according to the present disclosure.

### 1. Cell culture

Human embryonic kidney cells HEK293T (ATCC), human pancreatic cancer cells PANC-1, and MiaPaCa-2 (KCLB) were cultured in Dulbecco modified Eagle's medium (DMEM, Invitrogen) supplemented with 10% fetal bovine serum (FBS). Human pancreatic cancer cells AsPC-1 (KCLB) and BxPC-1 (ATCC) were cultured in Roswell Park Memorial Institute Medium (RPMI-1640, Invitrogen) supplemented with 10% fetal bovine serum (FBS). Cells were cultured in a wet incubator (85%) in the presence of 5% CO₂ at 37°C under sterile conditions. All experiments were performed on a sterile clean bench. When reaching 80% confluency, adhered cells were subcultured.

### 2. Antibodies and other reagents

Human polyclonal antibody against mesothelin (PA5-79697), Alexa Fluor 488 goat anti rabbit secondary antibody (A11008), and Alexa Fluor 594 goat anti rabbit secondary antibody (A11012) were purchased from Invitrogen. Cyclic CTILWSLTC (MSLN-pep, clone-1) was requested to AniGen for synthesis of a peptide linked via disulfide linkage using cysteine at the C- and N-terminus. Also for MSLN-pep-KLA synthesized by combining the apoptotic peptide KLAKLAKKLAKLAK and CTIL WSL TC (MSLN-pep, clone-1), the synthesis of a cyclic MSLN peptide via a disulfide linkage using cysteine at the C- and N-termini were requested to AniGen.

### 3. Isolation of expression vectors

The mesothelin-expressing plasmid pCMV6-AC-GFP vector purchased from Origene was transformed into E. coli DH5α (RH617) purchased from RBC Bioscience, and the DNA was isolated using a Mini-Prep Kit (Dokdo-Preparation) to obtain a high concentration of DNA for transient transfection.

### 4. Transfection

HEK293T cells were transiently transfected using Lipofectamine 3000 (Invitrogen) in 6-well plates according to the manufacturer's protocol. Prior to transfection, 2 × 10⁵ cells were cultured in 6-well plates for 24 hours. To transfect one well, 7.5 µl of Lipofectamine 3000 reagent was diluted in 250 µl of Opti-MEM (Invitrogen) reagent, followed by a reaction at room temperature for 5 minutes. 5 µg of mesothelin-expressing plasmid pCMV6-AC-GFP vector DNA was diluted in 250 µl of Opti-MEM (Invitrogen) reagent, followed by a reaction at room temperature for 5 minutes. The mixture of two solutions were then mixed and reacted at room temperature for 10 minutes (a total of 500 µl per well). The old medium of the cells was immediately removed, and a 500 µl of DNA-transfection reagent-mixture was added to the cells before transfer to the incubator. After 24 hours of culture, the medium was again replaced with a pre-warmed growth medium containing serum. Culture was performed for 48 hours, and the newly inserted protein was expressed in the cells.

### 5. Bio-panning of a phage peptide library for screening peptides that specifically bind to mesothelin

A phage peptide library based on the T7 415-1b phage vector, represented by CX7C (C, cysteine; X, any amino acid residue) was designed according to the manufacturer's instructions (Novagen, Madison, WI). The phage library has approximately 1 × 10⁹ plaque-forming unit (pfu). In other words, HEK-293T cells transfected with a mesothelin-expressing plasmid pCMV6-AC-GFP vector were dispensed in a 35 mm dish and cultured to meet a confluency of 60-70%. For transient transfection of HEK-293T cells, the cells were treated with Lipofectamine 3000 (Invitrogen) and cultured for 48 hours. First, phage libraries with 1 × 10⁹ plaque-forming unit (pfu) were cultured in untransfected HEK-293T cells at 4°C for 30 minutes. Afterwards, phage libraries that did not bind to untransfected HEK-293T cells were extracted and cultured in transfected HEK 293T cells at 4°C for 1 hour, and the phages bound to the cells were eluted with 500 µL of BL21 bacteria (OD: 1) culture medium at room temperature for 10 minutes. The eluate was used for titration, the remaining eluted phage clones were lysed in 10 ml of LB medium for amplification for the next cycle, and the process was repeated 5 times. The stepwise diluted product of the eluate was inoculated with E. coli cultured in LB medium for 2 hours at 37°C, followed by measurement of titer of the phage by counting the number of colonies.

In order to improve the selectivity of the phage clones specific to mesothelin-expressing cells, the phages were first cultured on untransfected cells, and the unbound phages were extracted and then cultured on transfected cells. In the present disclosure, a direct screening method was used to apply all phage clones in the next round as a method of amplification and phage concentration.

A total of 70 clones derived from transfected culture dishes in the third, fourth and fifth rounds (20 phage clones in the third round, 20 phage clones in the fourth round, and 20 phage clones in the fifth round) and 10 clones derived from untransfected culture dishes were sequenced to exclude unbound phage clones.

### 6. Analysis of migration

PANC-1 and AsPC-1 cell lines were cultured using DMEM and RPMI-1640 media with 10% FBS added respectively in the upper chamber of 24-transwell at a concentration of 2 × 10⁴ cells per well. After 24 hours, all the media in the upper chamber of 24-transwells were removed and replaced with DMEM and RPMI-1640 media without addition of 10% FBS to inhibit migratability of cell lines, so as to be in a starvation state. After 24 hours, the experimental group was added with 10% FBS in the lower chamber, and the control was added with 0% FBS in the lower chamber to observe migratability. 3% ACN, 200 uM control peptide (Ctrl-pep), MSLN-pep 25. For 50, 100, and 200uM groups, the media in the upper chamber of 24-Transwell were removed, each drug was treated by concentration, and 10% FBS was treated in the lower chamber. Each group was cultured at 37°C for 24 hours, and then the cells were photographed (original magnification, × 100) by concentration of each peptide. Cells were stained with crystal violet (0.1%), and the ImageJ, a computer program, was used for quantitative evaluation.

### 7. Analysis of invasion

PANC-1 and AsPC-1 cell lines were cultured using DMEM and RPMI-1640 media with 10% FBS added in 100 mm dish each at a concentration of 2 × 10⁶ cells per well. After 24 hours, all media in 100 mm dishes were removed and replaced with DMEM and RPMI-1640 media without addition of 10% FBS to inhibit migratability of cell lines so as to be in a starvation state. After 24 hours, 300 ug/mL of Matrigel was coated on the upper chamber of 24-Transwell. Afterwards, PANC-1 and AsPC-1 cell lines were cultured in the upper chamber at a concentration of 2 × 10⁴ cells in a 100 mm dish, 10% FBS was added to the lower chamber for the experimental group, and 0% FBS was added to the lower chamber for the control group to identify migratability. 3% ACN, 200uM control peptide (Ctrl-pep), MSLN-pep 25. For 50, 100, and 200uM groups, each cell line was cultured together in the upper chamber of the 24-Transwell, and 10% FBS was treated in the lower chamber. Each group was cultured at 37°C for 24 hours, and then the cells were photographed (original magnification, × 100) for each concentration of peptides. Cells were stained with crystal violet (0.1%), and Image J, a computer program, was used for quantitative evaluation.

### 8. Suppression of mesothelin expression using siRNA

Mesothelin-specific siRNA (M-siRNA) and negative control siRNA (C-siRNA) were purchased from Bioneer and transfected with Lipofectamine RNAiMAX transfection reagent (Invitrogen) into AsPC-1, a pancreatic cancer cell line. After 24 hours of transfection, the media were replaced with a medium containing serum, followed by culture for an additional 24 hours.

### 9. Analysis of internalization of FITC-labeled peptides using confocal scanning microscopy

To observe the intracellular distribution of the internalized FITC-labeled peptide MSLN-pep, pancreatic cancer cell lines AsPC-1 and PANC-1 cells (2.5 × 10⁵ cell counts) were dispensed on a 4-well chamber slide. After complete adhesion, the cell culture medium was replaced with a fresh medium containing FITC-labeled peptides (25µM, 50uM), culture was performed at 37°C for 1 hour, and the cells were washed with PBS 3 times for 3~5 minutes, followed by fixation (4% paraformaldehyde; PFA) for 5 minutes. Thereafter, washing with PBS was performed. After staining the nucleus with DAPI and mounting on a glass slide with a fading reagent, internalization of FITC-labeled MSLN-pep was observed under confocal microscopy.

### 10. Analysis of cytotoxicity

Pancreatic cancer cell line AsPC-1 cells (5 × 10³ cells/well in 96-well plates) were cultured in 1% serum medium with various concentrations of MSLN-pep peptide that specifically binds to mesothelin, NSSSVDK control peptide (Ctrl-pep), R7-KLA peptide that induces apoptosis, Abraxane, gemcitabine, and PBS at 37°C for 24 and 48 hours. Afterwards, CCK-8 Kit (Dojindo, Kumamoto, Japan) reagent was treated, and a reaction was carried out at 37°C for 2 hours to evaluate cytotoxicity.

### 11. Analysis of tumor homing of mouse peptides

Pancreatic cancer cell line AsPC-1 cells (1 × 10⁷ cells) were implanted under the skin of nude mice, left until the tumor size reaches about 100 mm³, and then intravenously injected with 100 µl of 1 mM peptides labeled with a TAMRA fluorescence reagent, followed by circulation for 6 hours. Tumors and organs (liver, lung, and kidney) were separated and treated with paraformaldehyde for fixation and preparation of cryosections. The prepared cryosections were observed under a fluorescence microscope.

### 12. Analysis of anticancer treatment effects on mice

Pancreatic cancer cell line AsPC-1 cells (2 × 10⁶ cells) were implanted under the skin of nude mice, left until the tumor size reaches about 100 mm³, and then administered as follows. MSLN-pep-KLA or Ctrl-pep-KLA was administered intravenously at a dose of 10 mg/kg three times a week for a total of 7 times. Gemcitabine was administered intraperitoneally at a dose of 10 mg/kg twice a week for a total of 7 doses. During chemotherapy, the size and weight of the tumor were measured, and then the tumor was removed to observe its size. In addition, after treatment, blood was collected to measure the levels of liver enzymes and renal function indicators in the serum.

### <Example 1> Expression of mesothelin by pancreatic cancer cell lines

In order to observe whether mesothelin is expressed in pancreatic cancer cells, pancreatic cancer cell lines AsPC-1, PANC-1, BxPC-3, and MiaPaCa-2 were cultured in a 4 well chamber and treated with mesothelin antibodies to detect expression of mesothelin in pancreatic cancer cell lines (FIG. 1A). Further, the resulting order from analysis of the expression level of mesothelin in pancreatic cancer cell lines via Western blot was found to be AsPC-1, BxPC-3, PANC-1, and MiaPaCa-2 (FIG. 1B). In addition to pancreatic cancer cell lines, the breast cancer cell lines MDA-MB-231 and MCF7 also showed less mesothelin expression than pancreatic cancer cell lines, but higher than the normal cell lines HEK-293T and MCF10A (FIG. 1C).

### <Example 2> Screening of mesothelin-binding peptides

To screen peptides specifically binding to mesothelin, the present inventors first prepared HEK-293T cells transiently transfected with a mesothelin-GFP plasmid. To detect mesothelin expression along with GFP expression, HEK-293T transfected cells were stained with mesothelin antibodies. As a result of observing mesothelin-GFP expression via immunofluorescence analysis, transfection was detected at each time point compared to untransfected HEK-293T cells (FIG. 2A). In order to screen mesothelin-specific peptides by direct screening methods, the present inventors identified the accumulative titers of all phage clones in each round, which was significantly increased compared to the untransfected titers, and it was found that the phage accumulative titers increased by 4.6 × 10¹ times per round for 5 rounds (FIG. 2B). In the third, fourth, and fifth rounds, 20 phage clones were randomly screened and sequenced with peptide-coding DNA inserts of the phage clones. Phage clones that bind to mesothelin-expressing cells with high specificity were screened for phage-binding ELISA, and several clones were found to bind in multiple expressing and non-expressing cell lines (FIGS. 3A and 3B). Compared to other phage clones, it was found that clone-1 binds to mesothelin in high degree through phage ELISA and phage immunofluorescence staining, showing the binding specificity and effect of the phage clone. The peptide sequence of the single clone is as follows. CTILWSLTC (hereinafter, MSLN-pep, clone-1; SEQ ID NO: 1).

### <Example 3> Binding of MSLN-pep to mesothelin-expressing pancreatic cancer cell lines

After treatment of 10 µM and 25 µM MSLN-peps to mesothelin-expressing pancreatic cancer cell lines AsPC-1, PANC-1, BxPC-3, and MiaPaCa-2, the result showed specific binding of MSLN-pep even at 10 µM with the further increase at 25 µM (FIG. 4A). As a result of treating 25 µM NSSSVDK (Ctrl-pep) non-specifically binding to 10 uM and 25 uM MSLN-pep to HEK-293T cells transiently transfected with mesothelin-GFP plasmids and untransfected HEK-293T, the derived results showed that the MSLN-pep bound only to the mesothelin-expressing portion while Ctrl-pep did not have specific binding (FIG. 4B). Taken together, it was found via immunofluorescence analysis that MSLN-pep specifically binds to mesothelin.

### <Example 4> Analysis of location of mesothelin and MSLN-pep binding site in mesothelin-expressing pancreatic cancer cell lines

Pancreatic cancer cell lines AsPC-1, PANC-1, and BxPC-3 were cultured in a 4-well chamber and then treated with mesothelin antibodies to determine the location where mesothelin is expressed. Then, as a result of detecting a mesothelin binding site by treating the TAMRA-labeled MSLN-pep, it was found by confocal microscopy that the binding took place at the same spot where the mesothelin is expressed (FIG. 5A).

### <Example 5> Analysis of binding of MSLN-pep in pancreatic cancer cell lines whose expression is suppressed by siRNA for mesothelin

After AsPC-1 cells were cultured for 24 hours, for suppression of mesothelin expression, transfection followed with C-siRNA, a non-specific siRNA, as well as M-siRNA, a mesothelin-specific siRNA. To detect the silencing of the mesothelin gene, the group was divided into one without treatment of siRNA, one with treatment of 100 nM C-siRNA, and one with treatment of 100 nM and 200 nM M-siRNA. Mesothelin antibody and MSLN-pep were then treated by 10 µM and 25 µM, respectively. Further, the results were identified by means of confocal microscopy via immunofluorescence analysis (FIG. 6A). The results showed that higher specific binding to mesothelin took place when the concentration of MSLN-pep was 25 uM compared to 10 µM, and the specific binding of mesothelin antibodies and MSLN-pep decreased with the decrease of mesothelin expression when the concentration of M-siRNA was 100 nM and 200 nM. Flow cytometry was performed to determine the expression level of mesothelin when siRNA was not treated to AsPC-1 cells and the expression level of mesothelin after C-siRNA and M-siRNA were treated, of which values were expressed in % binding with the mesothelin antibody and the mean fluorescence intensity (MFI). When treated with no siRNA and with 100 nM C-siRNA, binding with mesothelin antibodies was approximately 75%, and when treated with 100 nM and 200 nM M-siRNA, the binding significantly dropped to about 10%. As a result of performing flow cytometry to determine the difference between the binding of 25 uM MSLN-pep when siRNA was not treated to AsPC-1 cells expressing mesothelin and the binding of 25 uM MSLN-pep after treating with C-siRNA and M-siRNA, when treated with no siRNA or treated with 100 nM C-siRNA, the specific binding of 25 uM MSLN-pep was approximately 78%, and when treated with 100 nM and 200 nM M-siRNA, it was approximately 15%. This series of results reveal the degree of mesothelin expression and the reduction of mesothelin expression when M-siRNA is treated. In addition, when treated with M-siRNA, specific binding of 25uM MSLN-pep resulted in reduced specific binding as much as the reduced expression of mesothelin protein (FIG. 6B). In brief, it was determined that MSLN-pep specifically binds to cells expressing mesothelin.

### <Example 6> Analysis of internalization into cells after binding MSLN-pep to mesothelin-expressing cells

After pancreatic cancer cell lines PANC-1 and AsPC-1 were cultured in a 4-well chamber, 50 uM NSSSVDK control peptide and 25 µM and 50 µM mesothelin-specific binding peptides MSLN-pep were treated respectively and observed for 1 and 2 hours, and the result by confocal microscopy showed that 50 uM NSSSVDK did not bind to PANC-1 and AsPC-1, and, in the case of 25 uM and 50 uM MSLN-pep, internalization into cells occurs in proportion to the increase in the concentration (FIG. 7A).

### <Example 7> Inhibition of migratability and invasiveness of cells expressing mesothelin by MSLN-pep

After pancreatic cancer cell lines PANC-1 and AsPC-1 were cultured in 24-transwells, all cells were starved for 24 hours to inhibit cell growth, and then treatment was performed in divided groups of 10% FBS, 0% FBS, 3% ACN + 10% FBS, 200uM NSSSVDK + 10% FBS, 25uM MSLN-pep + 10% FBS, 50uM MSLN-pep + 10% FBS, 100uM MSLN-pep + 10% FBS, and 200uM MSLN-pep + 10% FBS. The results showed that migratability and invasiveness increased upon treatment of 10% FBS and decreased upon treatment of 0% FBS. There was no difference in the migratability and invasiveness in the treatment of 3% ACN + 10% FBS compared to treatment of 10% FBS, with no difference in the migratability and invasiveness in the treatment of 200uM NSSSVDK + 10% FBS and 25uM MSLN-pep + 10% FBS compared to treatment of 10% FBS. In the treatment of 50uM MSLN-pep + 10% FBS, 100uM MSLN-pep + 10% FBS, and 200uM MSLN-pep + 10% FBS, migratability and invasiveness were suppressed with increasing concentrations of MSLN-pep (FIG. 8A and FIG. 8B).

### <Example 8> Analysis of tumor homing of peptides in mice

Pancreatic cancer cell line AsPC-1 cells (1 × 10⁷ cells) were implanted under the skin of nude mice, left until the tumor size reached about 100 mm³, and then intravenously injected with MSLN-pep labeled with the TAMRA fluorescence reagent and control peptide (Ctrl-pep), followed by circulation for 6 hours. The tumor and control organs were separated, and cryosections were prepared, followed by observation under a fluorescence microscope.

As a result, there was high expression levels of mesothelin in the tumor tissue, and the target of MSLN-pep was observed at a higher level compared to the control (FIG. 9). On the other hand, there was little expression of mesothelin in control organs such as liver, lungs, and kidneys, and the control as well as targets of MSLN-pep were barely observed. These results show that the peptide of the present disclosure is effectively targeted to tumor tissue in vivo, indicating that it may be used for selective delivery of anticancer drugs as well as diagnosis of cancer through imaging when linked to contrasts.

### <Example 9> Analysis of cytotoxicity of MSLN-pep and apoptotic peptide-linked peptides

To determine whether there is cytotoxicity when MSLN-pep binds specifically to mesothelin, the peptide was treated to the pancreatic cancer cell line AsPC-1 at concentrations of 0, 25, 50, 100, and 200 µM, and cell viability was checked for 24 and 48 hours. The results showed that the cell viability was about 80% upon 200 uM treatment if 100% cell viability is set with no treatment of MSLN-pep. In other words, even when high concentrations of MSLN-pep were treated, the cytotoxicity of the peptide alone was low (FIG. 10A). Abraxane and gemcitabine, anticancer agents used in the treatment of pancreatic cancer, were treated to AsPC-1, a pancreatic cancer cell line, at 0, 2, 4, 6, 8, and 10 ug/mL (log scale, ug/mL), and, as a result of checking the cell viability 24 hours later, the cell viability upon treatment of 10 ug/mL was less than or equal to 40% as the cytotoxicity increased with the increasing concentration, with the IC50 value of 7.1 ug/mL for Abraxane and 3.3 ug/mL for Gemcitabine, indicating that the cytotoxicity of gemcitabine is slightly stronger than that of Abraxane (FIG. 10B).

Based on the results that there is no cytotoxicity in MSLN-pep and internalization into cells, it was investigated whether the linkage of the apoptotic peptide KLAKLAKKLAKLAK to MSLN-pep (hereinafter referred to as MSLN-pep-KLA) leads to effective apoptosis. After dividing into groups treated with PBS, R7-KLA which causes non-specific apoptosis to all cells, Ctrl-pep-KLA, and MSLN-pep-KLA, the pancreatic cancer cell line AsPC-1 was treated in concentrations of 0, 6.25, 12.5, 25, 50, 100, and 200 uM, and, as a result of checking the cell viability after 24 hours, the cell viability was the lowest when treated with R7-KLA at all concentrations, while the cell viability was the second lowest when treated with MSLN-pep-KLA that specifically binds to mesothelin. When Ctrl-pep-KLA was treated, there was no significant change in cell viability even when the concentration increased, but cell viability decreased sharply at 200 uM. From these results, it may be seen that MSLN-pep-KLA reduces cell viability by inducing apoptosis by binding specifically with mesothelin, and the IC50 value was 33 uM (FIG. 10C).

Next, investigation was conducted on the cytotoxicity in the co-treatment of gemcitabine and MSLN-pep-KLA. As a result of checking the cell viability by dividing into groups of pancreatic cancer cell line AsPC-1 subjected to the sole or co-treatment in 1/2 concentrations of 3.3 ug/mL as the IC50 value of gemcitabine and 33 uM as the IC50 value of MSLN-pep-KLA and groups subjected to sole or co-treatment in the IC50 value, co-treatment with gemcitabine and MSLN-pep-KLA in the IC50 value showed the strongest cytotoxicity (FIG. 10D). Interestingly, co-treatment of gemcitabine with MSLN-pep-KLA at 1/2 the IC50 value showed the same level of cytotoxicity as the case that gemcitabine is treated in the IC50 value (FIG. 10D). A similar pattern of cytotoxicity was seen in PANC-2, a mouse pancreatic cancer cell line expressing mouse mesothelin with approximately 70% homology to human mesothelin, but with relatively weaker results in all groups compared to PANC-1 cells (FIG. 10E). In addition, the normal cell line HEK-293T expressing little mesothelin showed little cytotoxicity even in the treatment with gemcitabine and MSLN-pep-KLA (FIG. 10F). In the above examples, MSLN-pep-KLA has specific cytotoxicity for cancer cells expressing mesothelin, and by combining with gemcitabine, an existing anticancer agent that has strong cytotoxicity but is limited in use due to strong side effects, it has been shown that it may have a similar level of anticancer effect while lowering the dosage of the drug.

### <Example 10> Analysis of the cancer growth inhibitory effect of MSLN-pep and apoptotic peptide-linked peptides in a mouse tumor model

To investigate the anticancer treatment effect of MSLN-pep-KLA, AsPC-1, a pancreatic cancer cell line, was implanted under the skin of nude mice to develop tumors, and then 10 mg/kg of MSLN-pep-KLA was treated alone or in combination with 10 mg/kg of gemcitabine. Ctrl-pep-KLA was used as a control. As a result, MSLN-pep-KLA alone showed a similar level of cancer growth inhibitory effect to gemcitabine and showed superior anticancer effect when co-administered (FIG. 11A). Almost no change was observed in the body weight for all administration groups (FIG. 11B). After treatment is completed, the tumor was isolated, and actual size thereof was observed, resulting in a pattern similar as in FIG. 11A (FIG. 11C). On the other hand, as a result of investigating liver and kidney toxicity after treatment, the levels of the liver enzymes ALT (FIG. 12A), AST (FIG. 12B), and ALP (FIG. 12C) were within the normal range, and the levels of CRE (FIG. 12D) and BUN (FIG. 12E), which indicate renal functions, barely go beyond the normal range.

While a specific part of the present disclosure has been described in detail above, it is clear for those skilled in the art that this specific description is merely preferred example embodiments, and the scope of the present disclosure is not limited thereby. In other words, the substantial scope of the present disclosure is defined by the appended claims and their equivalents.

## Claims

1. A peptide for specifically binding to mesothelin, comprising an amino acid sequence represented by SEQ ID NO: 1

2. A polynucleotide encoding the peptide according to claim 1.

3. A recombinant vector comprising the polynucleotide according to claim 2.

4. A transformant transformed with the recombinant vector according to claim 3 (except for humans).

5. A composition for diagnosing cancer, comprising the peptide according to claim 1 as an active ingredient.

6. The composition of claim 5, wherein the cancer is one with a high expression level of mesothelin.

7. The composition of claim 6, wherein the cancer is any one selected from the group consisting of mesothelioma, ovarian cancer, pancreatic cancer, lung cancer, cervical cancer, biliary tract cancer, breast cancer, head and neck cancer, and esophageal cancer.

8. A pharmaceutical composition for inhibiting cancer metastasis, comprising the peptide according to claim 1 as an active ingredient.

9. The pharmaceutical composition of claim 8, wherein the peptide binds specifically to mesothelin, is internalized into cancer cells, and inhibits invasion and migration of cancer cells.

10. A composition for drug delivery, comprising the peptide according to claim 1 as an active ingredient.

11. The composition of claim 10, wherein the drug is a peptide drug or an anticancer agent.

12. The composition of claim 11, wherein the peptide drug is a cytotoxic peptide with apoptosis or necrosis-inducing activity.

13. The composition of claim 11, wherein the anticancer agent is any one or more selected from the group consisting of gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

14. A fusion peptide in which the peptide according to claim 1 and an apoptotic peptide having an amino acid sequence represented by SEQ ID NO: 2 are linked.

15. A pharmaceutical composition for preventing or treating cancer, comprising the fusion peptide according to claim 14 as an active ingredient.

16. A pharmaceutical composition for preventing or treating cancer, comprising the fusion peptide according to claim 14 and an anticancer agent as active ingredients.

17. The pharmaceutical composition of claim 16, wherein the anticancer agent is any one or more selected from the group consisting of gemcitabine, crizotinib, doxorubicin, paclitaxel, vincristine, daunorubicin, vinblastine, actinomycin-D, docetaxel, etoposide, teniposide, bisantrene, homoharringtonine, Gleevec (STI-571), cisplatin, 5-fluorouracil, adriamycin, methotrexate, busulfan, chlorambucil, cyclophosphamide, melphalan, nitrogen mustard, and nitrosourea.

18. The pharmaceutical composition of any one of claim 15 to claim 17, wherein the cancer is one with a high expression level of mesothelin.

19. The pharmaceutical composition of claim 18, wherein the cancer is any one or more selected from the group consisting of mesothelioma, ovarian cancer, pancreatic cancer, lung cancer, cervical cancer, biliary tract cancer, breast cancer, head and neck cancer, and esophageal cancer.

20. A composition for cancer cell imaging, comprising the peptide according to claim 1 as an active ingredient.

21. The composition of claim 20, wherein the peptide is labeled with any one selected from the group consisting of chromogenic enzymes, radioisotopes, chromophores, luminescent substances, fluorescers, magnetic resonance imaging substances, superparamagnetic particles, and ultrasuper paramagnetic particles.
